# FASCICULE DE BREVET EUROPEEN

(11) **EP 3 268 463 B1**
(45) Date de publication et mention de la délivrance du brevet: **19.12.2018**
(21) Numéro de dépôt: 16712964.2
(22) Date de dépôt: 09.03.2016
(51) Int. Cl.: C12N 1/20, C12Q 1/04

(54) **MILIEU ET PROCEDE DE CULTURE DES MYCOBACTERIES COMPRENANT DU SERUM D'AGNEAU**
MEDIUM UND VERFAHREN ZUR ZÜCHTUNG VON MYKOBAKTERIEN MIT LAMMSERUM
MEDIUM AND METHOD FOR CULTURING MYCOBACTERIA COMPRISING LAMB SERUM

(30) Priorité: 11.03.2015 FR 1552007
(43) Date de publication de la demande: 17.01.2018
(73) Titulaire: Fondation Méditerranée Infection, 13005 Marseille (FR); Université d'Aix-Marseille, 13284 Marseille Cedex 07 (FR)
(72) Inventeur: RAOULT, Didier, 13008 Marseille (FR); DRANCOURT, Michel, 13012 Marseille (FR); ASMAR, Shady, Meten Mont-Liban (LB); CHATELLIER, Sonia, 01500 Amberieu en Bugey (FR)
(74) Mandataire: Cabinet Beau de Loménie
(86) Numéro de dépôt international: PCT/FR2016/050539
(87) Numéro de publication internationale: WO 2016/142626

(56) Documents cités:
- WO-A1-2010/063911
- US-A- 3 360 440
- A. Y. COBAN ET AL: "Evaluation of Agar-Based Medium with Sheep Sera for Testing of Drug Susceptibility of Mycobacterium tuberculosis to Isoniazid, Rifampin, Ethambutol, and Streptomycin", JOURNAL OF CLINICAL MICROBIOLOGY, vol. 51, no. 12, 16 octobre 2013 (2013-10-16), pages 4243-4245, XP055239607, US ISSN: 0095-1137, DOI: 10.1128/JCM.01947-13
- anonymous: "Lamb Serum", Thermo Fisher Scientific , 1 janvier 2015 (2015-01-01), XP002752814, Extrait de l'Internet: URL:https://www.thermofisher.com/nl/en/hom e/life-science/cell-culture/mammalian-cell -culture/fbs/other-sera/lamb-serum.html [extrait le 2016-01-08]
- LASZLO KATO: "Cholesterol, a Factor Which is Required for Growth of Mycobacteria from Leprous Tissues", INTERNATIONAL JOURNAL OF LEPROSY AND OTHER MYCOBACTERIAL DISEASES., vol. 46, no. 2, 1 janvier 1978 (1978-01-01), pages 133-143, XP055240005, XX ISSN: 0148-916X
- ASMAR S ET AL: "A novel solid medium for culturing mycobacterium tuberculosis isolates from clinical specimens", JOURNAL OF CLINICAL MICROBIOLOGY 20150801 AMERICAN SOCIETY FOR MICROBIOLOGY USA, vol. 53, no. 8, 1 août 2015 (2015-08-01), pages 2566-2569, XP002752815, ISSN: 0095-1137

## Description

La présente invention concerne un nouveau milieu de culture des mycobactéries, en particulier des mycobactéries du complexe *Mycobacterium tuberculosis,* permettant de réduire de façon significative les délais d'isolement par culture et donc le délai de diagnostic des mycobactérioses, en particulier de la tuberculose.

Les mycobactéries sont des bactéries classées dans le phylum des *Actinobacteria* par le séquençage du gène 16S ARNr et par les analyses dites "multilocus phylogeny" [Mignard S, Flandrois JP. A seven-gene, multilocus, genus-wide approach to the phylogeny of mycobacteria using supertrees. Int J Syst Evol Microbiol. 2008;58:1432-41] et caractérisées par la présence d'acides mycoliques dans leur paroi, ce qui leur confère une affinité tinctoriale particulière mise en évidence notamment par la coloration de Ziehl-Neelsen, et par un chromosome à haut G + C % > 60 % [Pfyffer GE. Mycobacterium : général characteristics, laboratory detection, in staning procedures. In : Murray Pr, Baron EJ, Jorgensen JH, Landry ML, Pfaller MA. Manual of Clinical Microbiology 9ème Eds. Amercian Society for Microbiology, Washington DC; 2007, pp. 543-572]. Le genre bactérien *Mycobacterium* comporte plus de cent espèces, dont des espèces environnementales isolées d'environnements inertes (sol, eau), des espèces associées aux animaux et une espèce essentiellement associée à l'homme, *Mycobacterium leprae,* agent de la lèpre [Cole S et al. Massive gene decay in the leprosy bacillus. Nature 2001;409:1007-11]. Certaines espèces environnementales sont responsables d'infections opportunistes chez l'homme (les espèces du complexe *Mycobacterium avium* et du complexe *Mycobacterium abscessus* par exemple) et certaines espèces sont responsables de zoonoses, en particulier certaines espèces de complexe *Mycobacterium tuberculosis,* responsables de la tuberculose [Ghodbane R, Drancourt M. Non-human sources of Mycobacterium tuberculosis. Tuberculosis (Edinb). 2013 Nov;93(6);589-95].

Le diagnostic des mycobactérioses repose sur l'isolement et la culture de l'une des espèces du genre *Mycobacterium* à partir d'un prélèvement clinique réalisé chez l'homme ou chez l'animal. De ce point de vue, le genre *Mycobacterium* comporte une espèce non-cultivable en milieu axénique (*Mycobacterium leprae*), des espèces de croissance rapide (*Mycobacterium fortuitum, Mycobacterium abscessus*) donnant des colonies visibles en moins de sept jours de culture et des espèces à croissance lente donnant des colonies visibles en plus de sept jours de culture, soit en pratique de routine, entre 3 et 8 semaines de culture, notamment avec des milieux Middlebrook. En médecine humaine, les espèces du complexe *Mycobacterium avium* sont détectables après 10-20 jours de culture et les espèces du complexe *Mycobacterium tuberculosis* requièrent 10-100 organismes viables par ml de prélèvement et 6-8 semaines de culture pour obtenir 100% de prélèvements positifs [Colebunders R, Bastian I. A review of the diagnosis and treatment of smear-negative pulmonary tuberculosis. Int J Tuberc Lung Dis. 2000;4:97-107]. Etant donné l'importance numérique et la gravité des cas de tuberculose humaine, c'est sur le diagnostic de la tuberculose que les améliorations des techniques de laboratoire sont particulièrement sensibles. En effet, la tuberculose est une maladie infectieuse de l'homme et des animaux due à l'une des neuf espèces du complexe *Mycobacterium tuberculosis. Mycobacterium tuberculosis, Mycobacterium bovis* (et les souches BCG qui en sont dérivées), *Mycobacterium africanum, Mycobacterium canettii, Mycobacterium caprae, Mycobacterium microti, Mycobacterium pinnipedii* [Dye C. et al. Prospects for worlwide tuberculosis control under the WHO DOTS strategy. Lancet 1998; 352:1886-91; Bos KI, Harkins KM, Herbig A, Coscolla M, Weber N, Comas I, Forrest SA, Bryant JM, Harris SR, Schuenemann VJ, Campbell TJ, Majander K, Wilbur AK, Guichon RA, Wolfe Steadman DL, Cook DC, Niemann S, Behr MA, Zumarraga M, Bastida R, Huson D, Nieselt K, Young D, Parkhill J, Buikstra JE, Gagneux S, Stone AC, Krause J. Pre-Columbian mycobacterial genomes reveal seals as a source of New World human tuberculosis. Nature 2014;514:494-7], *Mycobacterium mungi* [Alexander KA, Laver PN, Michel AL, Williams M, van Helden PD, Warren RM, Gey van Pittius NC. Novel Mycobacterium tuberculosis complex pathogen, M. mungi. Emerg Infect Dis. 2010;16:1296-9] et *Mycobacterium suricattae* [Parsons SD, Drewe JA, Gey van Pittius NC, Warren RM, van Helden PD. Novel cause of tuberculosis in meerkats, South Africa. Emerg Infect Dis. 2013;19:2004-7]. L'Organisation Mondiale de la Santé (OMS) a estimé que la tuberculose a été responsable de 9 millions de nouveaux cas et de 1,5 millions de décès en 2013 [World Health Organization. 2014. Global tuberculosis report 2014: Geneva : World Health Organization]. Ces chiffres soulignent l'importance qu'il y a à optimiser le diagnostic microbiologique de la tuberculose afin d'améliorer la prise en charge médicale des patients et de leur entourage.

L'isolement et la culture des mycobactéries du complexe *Mycobacterium tuberculosis* sont réalisées à partir de prélèvements cliniques obtenus chez un patient présentant des signes et des symptômes évocateurs de la tuberculose. La forme clinique la plus fréquente, qui est aussi la seule forme clinique contagieuse, est la tuberculose pulmonaire qui est diagnostiquée par l'isolement et la culture d'une mycobactérie du complexe *Mycobacterium tuberculosis* à partir d'un prélèvement respiratoire tel que l'expectoration, l'aspiration bronchique, le liquide de lavage broncho-alvéolaire obtenu sur bronchoscopie, voire la biopsie pulmonaire. Chez les patients ne produisant pas d'expectoration, les selles peuvent être utilisées comme une alternative pour l'isolement et la culture des mycobactéries du complexe *Mycobacterium tuberculosis* en cas de tuberculose pulmonaire [El Khéchine A, Henry M, Raoult D, Drancourt M. Détection of Mycobacterium tuberculosis complex organisms in the stools of patients with pulmonary tuberculosis. Microbiology 2009;155:2384-9]. Il existe d'autres formes cliniques de tuberculose, en particulier la tuberculose ganglionnaire, mais également les tuberculoses osseuses (Mal de Pott) ainsi que les tuberculoses digestives. En fonction de la forme clinique, différents prélèvements cliniques peuvent être adressés au laboratoire pour isoler et cultiver les mycobactéries du complexe *Mycobacterium tuberculosis* et diagnostiquer les formes extrapulmonaires.

Pour l'isolement et la culture des mycobactéries du complexe *Mycobacterium tuberculosis,* on dispose de milieux solides, de milieux liquides, et de milieux biphasiques comportant une phase liquide et une phase solide. Les milieux solides sont fabriqués à base de gélose ou d'agar. Les milieux contenant de l'oeuf entier sont l'utilisation très courante et le milieu le plus utilisé est le milieu de Löwenstein Jensen. Ce milieu, comme les autres milieux contenant de l'oeuf, contient du vert malachite qui participe à inhiber la croissance des microorganismes contaminants. Plusieurs formulations contenant des concentrations variables de vert malachite ont été proposées avec comme résultat constant qu'une diminution de la concentration de vert malachite augmente le ratio de contamination du milieu et une augmentation de la concentration de vert malachite tend à diminuer l'isolement et la culture des mycobactéries du groupe tuberculeux. Une deuxième catégorie de milieux solides sont les milieux à l'agar en particulier le milieu Middlebrook 7H10 et le milieu 7H11 (milieu 7H10 plus 0,1% de caséine hydrolysée). Le milieu de Middlebrook contient 2% de glycérol, qui facilite la culture des mycobactéries du complexe *Mycobacterium avium.* Les milieux liquides correspondent essentiellement au milieu Middlebrook 7H9.

Cependant, l'isolement des mycobactéries du complexe *Mycobacterium tuberculosis* est lent puisque la totalité des souches des différentes espèces du complexe *Mycobacterium tuberculosis* est isolée dans un délai compris entre 6 et 8 semaines. Tout gain de temps sur ce délai représente donc une amélioration significative du diagnostic de laboratoire de la tuberculose et les autres infections à mycobactérie.

Dans le brevet EP 2 364 357, on a décrit une formulation de milieux de culture permettant l'isolement plus rapide des mycobactéries du complexe *Mycobacterium tuberculosis* et des autres mycobactéries, notamment permettant une détection et identification détectable visuellement à l'oeil nu en moins de 15 jours, voire même en 10 jours, à partir d'échantillons de prélèvement cliniques. Cette formulation de EP 2 364 357 permet l'isolement des mycobactéries avec la capacité de détecter visuellement à l'oeil nu les mycobactéries en croissance, aussi bien en milieu liquide adapté aux automates de détection qu'en milieu solide pour une détection manuelle, (notamment les compositions des exemples 1B (milieu liquide) et 2B (milieu solide) cette dernière étant ci-après dénommée MOD4). Ces formulations du fait de la présence de lécithine, sont, de surcroît, compatibles avec une décontamination à la chlorhexidine dans le cas de prélèvements cliniques pouvant comprendre des bactéries de la flore commensale risquant d'inhiber la croissance des mycobactéries [Ghodbane R, Raoult D, Drancourt M. Dramatic réduction of culture time of Mycobacterium tuberculosis. Sci Rep. 2014;4:4236].

Le délai diagnostique basé sur la culture dépend non seulement des qualités propres du milieu de culture -ici la présence de sérum d'agneau dans le milieu de culture selon l'invention, mais également, de la capacité à détecter les mycobactéries du complexe *Mycobacterium tuberculosis* en croissance. Plus particulièrement lors de l'utilisation des milieux solides, il s'agit de la capacité à détecter le plus rapidement possible l'apparition des colonies de *Mycobacterium tuberculosis.* En routine, les colonies sont détectées par l'observation macroscopique du milieu de culture à l'oeil nu correspondant à la détection de colonies de *Mycobacterium tuberculosis* de quelques millimètres ce qui requiert la présence d'un contraste entre la couleur des colonies et celle du milieu de culture.

Plus précisément, dans EP 2 364 357, les milieux de culture de mycobactéries, comprennent des facteurs de croissance de mycobactéries et, de préférence, des antibiotiques sans activité à l'égard des mycobactéries, caractérisés en ce qu'ils comprennent les composants additionnels suivants :
- de la lécithine, et
- du sang défibriné, et
- du sérum de veau foetal décomplémenté.

Cependant, la mise en oeuvre de sang, rend ces formulations difficiles à manipuler lors de sa fabrication industrielle et limite le délai de péremption dudit milieu car le sang doit être mis en oeuvre dans un délai inférieur à 24h et conservé à 4°C pour rester frais. En outre, le sang doit être prélevé en présence d'anticoagulants pour éviter sa coagulation ces anticoagulants pouvant avoir un effet négatif sur la croissance des mycobactéries.

C'est pourquoi, il existe un intérêt à mettre au point un milieu conservant les caractéristiques et performances des milieux décrit ci-dessus, mais ne comportant pas de sang selon le but de la présente invention.

Les inventeurs ont découvert qu'en remplaçant le sérum de veau foetal par du sérum d'agneau combiné à un colorant synthétique sans activité contre les mycobactéries, on obtient, en l'absence de sang, des résultats de croissance détectable à l'oeil nu, au moins identique voire amélioré permettant l'isolement rapide des mycobactéries du complexe *Mycobacterium tuberculosis* et des autres mycobactéries, notamment permettant une détection et identification détectable visuellement à l'oeil nu en moins de 15 jours, voire même en 10 jours, à partir d'échantillons de prélèvement cliniques. Cette combinaison sérum d'agneau combiné à un colorant synthétique compense les propriétés du sang en termes d'élément nutritif pour la croissance des mycobactéries et d'agent contrastant pour la capacité de détection visuelle à l'oeil nu. Cet agent contrastant permet de repérer les colonies à l'oeil nu pour ensuite les isoler et analyser le cas échéant par coloration de Ziehl-Neelsen ou de Kinyoun ou par spectrométrie de masse de type maldi tof comme décrit ci-après.

Plus précisément, la présente invention fournit un milieu de culture de mycobactéries apte à permettre la culture de mycobactéries à partir d'un échantillon de prélèvement clinique, comprenant de la lécithine, des facteurs de croissance de mycobactéries et, de préférence, au moins un composé antibiotique sans activité à l'égard des mycobactéries, et au moins un colorant sans activité antimycobactérienne, qu'il ne comprend pas de sang et comprend en outre au moins les composants additionnels suivants :
- du sérum d'agneau, de préférence décomplémenté, et
- au moins un colorant rouge sans activité contre les mycobactéries, permettant de contraster la couleur du milieu de culture par rapport aux colonies de mycobactéries.

Le sérum d'agneau contient moins d'immunoglobuline (d'anticorps et de facteurs inflammatoires) qu'un sérum de mouton et est plus performant en termes de culture de mycobactéries dans le cadre d'un procédé selon la présente invention à partir d'un prélèvement clinique. Il y a lieu de noter que la mise en oeuvre d'un milieu de culture pour l'isolement et la culture d'une souche de mycobactéries à partir d'un prélèvement clinique requière des propriétés plus exigeantes que les propriétés requises d'un milieu de culture pour des souches déjà isolées et établies en culture notamment dans le cadre des tests de susceptibilité aux antibiotiques pour antibiogramme, dans la mesure où la population bactérienne hétérogène présente dans un prélèvement clinique implique un processus de sélection de souches qui doivent s'adapter au milieu de culture.

De préférence, le sérum d'agneau est compris dans une proportion en volume de 2,5 à 25%, de préférence d'au moins 15%.

Le sérum d'agneau est de préférence décomplémenté, c'est-à-dire qu'on lui a retiré, de façon connue, l'ensemble des protéines appelées "complément sérique" par chauffage, notamment à 56°C pendant une heure, ce complément sérique pouvant avoir une activité antibactérienne.

Dans le milieu selon l'invention, en l'absence d'agent contrastant on différentie très difficilement la couleur crème des colonies par rapport à la couleur jaune clair du milieu de par la présence de lécithine et autres. En l'absence d'agent contrastant, il est nécessaire d'utiliser un instrument de type microscope inversé à faible grossissement permettant d'observer des colonies d'une taille d'environ un millimètre. Avantageusement encore, la détection des colonies peut se faire par autofluorescence des mycobactéries du complexe *Mycobacterium tuberculosis* éclairées sous une lumière ultraviolette et d'une longueur appropriée, permet de détecter des colonies d'une taille comprise entre 0,25 et 1 mm.

De préférence, le colorant est un colorant de couleur rouge à une concentration de 10 à 100 mg/L.

De préférence, le colorant de couleur rouge est choisi parmi l'azorubine et le rouge de Ponceau 4R de préférence encore à une concentration de 30 à 50 mg/L. Comme montré plus loin dans les exemples, ces colorants n'affectent pas la croissance des mycobactéries et présentent une grande stabilité dans le temps, notamment d'au moins 5 semaines.

De préférence encore, le colorant est un mélange d'azurobine et de rouge de Ponceau 4R de préférence encore dans une proportion en concentrations de 50/30.

De préférence encore, le colorant est un mélange d'azorubine à 50 mg/L et rouge Ponceau 4R à 30 mg/L.

Plus particulièrement, le milieu de culture de mycobactéries selon l'invention est constitué d'un milieu de culture de base, connu pour la culture des mycobactéries, comprenant notamment des sels minéraux, sucres, acides aminés, protéines et vitamines, ledit milieu de culture de base étant supplémenté par lesdits composants additionnels mentionnés ci-dessus.

De préférence, le milieu de culture de mycobactéries selon l'invention contient au moins un composé antioxydant, de préférence l'acide ascorbique de préférence à une concentration d'au moins 0.1 g/L. Ce composé antioxydant permet de s'affranchir du contrôle de la tension en oxygène inférieure à 5% et de réaliser une culture en atmosphère de 5%CO2 voire à l'air libre et atmosphère ambiante à la même température de 37°C.

Plus particulièrement, la lécithine est comprise dans une proportion pondérale de 0,1 à 5%, de préférence 0,5 à 1%.

Plus particulièrement encore, la lécithine est de la lécithine de jaune d'oeuf.

Plus particulièrement, un milieu de culture de mycobactéries selon l'invention comprend un milieu de culture de mycobactéries de base comprenant, outre de l'eau distillée, les composants suivants : sulfate d'ammonium, sulfate de magnésium, sulfate de cuivre, sulfate de zinc, citrate de sodium, citrate d'ammonium ferrique, chlorure de sodium, chlorure de calcium, phosphate monopotassique, phosphate disodique, acide L-glutamique, biotine et pyridoxine. Ces composants sont, notamment, les composants contenus dans les milieux de culture de mycobactéries dénommés milieux Middlebrook.

En pratique, ce milieu de culture se présente initialement sous forme d'un lyophilisat desdits composants listés ci-dessus, destiné à être dilué dans de l'eau distillée pour former le milieu de culture selon l'invention.

Plus particulièrement encore, le composé antibiotique sans activité antimycobactérienne est pris dans le groupe consistué par les polymyxines, l'acide nalidixique, le triméthoprime, l'azlocilline et la vancomycine. Avantageusement, le milieu de culture comporte, en outre, un antifongique qui est de préférence l'amphotéricine B.

L'ensemble constitué des antibiotiques et antifungiques cités ci-dessus, à l'exception de la vancomycine, constitue l'ensemble dénommé, connu de l'homme de l'art sous l'appellation PANTA.

Ledit milieu de culture est avantageusement un milieu de culture solide contenant un produit gélifiant choisi de préférence parmi les géloses et agar, de préférence dans une proportion pondérale de 0,5 à 5%, de préférence encore de 1 à 2%.

De manière préférentielle, il s'agit d'un milieu solide de type Middlebrook de référence 7H10, additionné avec ledit produit gélifiant et des facteurs de croissance de mycobactéries additionnels suivants : acide oléique, albumine bovine, de préférence la fraction V de l'albumine bovine, dextrose, catalase et glycérol. Le vert de malachite, contenu dans le milieu 7H10, est un inhibiteur de croissance des bactéries autres que mycobactéries. Les facteurs de croissance de mycobactéries additionnels ci-dessus, autres que le glycérol, sont connus sous la dénomination facteurs OADC.

La présente invention fournit également un procédé de détection par culture d'une mycobactérie à l'aide d'un milieu de culture de mycobactéries selon l'invention consistant à incuber un échantillon de prélèvement clinique pouvant contenir une mycobactérie, dans ledit milieu de culture de mycobactéries, à une température de 30 à 37°C, appropriée pour la culture de l'espèce de mycobactérie contenue dans ledit échantillon.

La présente invention fournit par ailleurs un procédé de détermination de la viabilité d'une mycobactérie préalablement détectée dans un échantillon de prélèvement clinique par un procédé approprié tel que, de façon non exhaustive, un procédé d'observation microscopique, un procédé de détection d'une séquence d'ADN, ou un procédé de détection antigénique.

La présente invention fournit enfin un procédé de détermination de la sensibilité et de la résistance d'une mycobactérie à des antibiotiques ou de façon plus générale, toute substance pouvant être mise en contact avec la mycobactérie en culture dans le milieu selon invention.

La plupart des mycobactéries est cultivée à 37°C. Toutefois, certaines mycobactéries, telles que *Mycobacterium marinum, Mycobacterium haemophilum, Mycobacterium ulcerans,* sont cultivées à une température de 28°C à 30°C. *Mycobacterium szulgai* peut être cultivée entre 25°C et 37°C, *Mycobacterium conspicuum* est cultivée entre 22°C et 31°C. La température optimale de croissance de *Mycobacterium xenopi* et de *Mycobacterium shimoidei* est de 45°C. Les bactéries *Mycobacterium genavense, Mycobacterium kansasii, Mycobacterium fortuitum,* les bactéries du complexe *Mycobacterium avium,* à savoir *Mycobacterium avium, Mycobacterium intracellulare, Mycobacterium chimaera* et *Mycobacterium colombiense* sont cultivées à 37°C.

Plus particulièrement, dans un procédé de culture d'une mycobactérie selon l'invention, on cultive un échantillon de prélèvement clinique contenant une bactérie du complexe *Mycobacterium tuberculosis* à une température de 37°C dans un dit milieu de culture de mycobactéries.

Un procédé de culture de mycobactéries selon l'invention peut également concerner des mycobactéries hors complexe *Mycobacterium tuberculosis,* telles que celles citées ci-dessus, notamment *Mycobacterium marinum,* les espèces du complexe *Mycobacterium avium, Mycobacterium haemophilum, Mycobacterium xenopi,* les espèces du complexe *Mycobacterium abscessus, Mycobacterium genavense, Mycobacterium kansasii, Mycobacterium ulcerans* et *Mycobacterium fortuitum.*

On entend ici par "bactéries du complexe *Mycobacterium tuberculosis",* les bactéries des espèces *Mycobacterium tuberculosis, Mycobacterium bovis* et ses clones ou sous-espèces BCG, *Mycobacterium africanum, Mycobacterium canettii, Mycobacterium caprae, Mycobacterium microti, Mycobacterium pinnipedii, Mycobacterium mungi* et *Mycobacterium suricattae.*

Avantageusement, dans un procédé de culture d'une mycobactérie selon l'invention, on réalise les quatre étapes suivantes:
- on effectue la culture d'un échantillon de prélèvement clinique pouvant contenir des mycobactéries jusqu'à ce qu'une croissance de bactéries soit détectable, et
- on identifie que la bactérie détectée est une bactérie du genre mycobactérie par un test de coloration, de préférence un test de coloration de Ziehl-Neelsen ou de Kinyoun, et
- le cas échéant, on identifie l'espèce de ladite mycobactérie par des moyens d'analyse moléculaire, de préférence par analyse de la masse moléculaire des protéines bactériennes par spectrométrie de masse.

Les moyens d'analyse moléculaire peuvent être des moyens d'analyse des protéines bactériennes, notamment par détermination de leur masse moléculaire par spectrométrie de masse. Toutefois, alternativement, on pourra avoir recours aux méthodes classiques d'identification moléculaire du génome (ADN ou ARN) avec des sondes et/ou amorces d'amplification spécifiques des différentes espèces de mycobactéries, notamment telles que décrites dans la demande WO 2008/050064. En particulier, pour la détection de mycobactéries du complexe *Mycobacterium tuberculosis,* on peut avoir recours à un système appelé multi spacer sequence typing (abrégé ci-après en MST) constitué d'une série de fragments d'acides nucléiques de zones intergéniques non codantes du génome des bactéries du complexe *Mycobacterium tuberculosis,* lesdits fragments constituant des marqueurs génétiques permettant l'identification des différentes espèces du complexe *Mycobacterium tuberculosis* et le génotypage des isolats d'une même espèce du complexe *Mycobacterium tuberculosis* et, notamment de l'espèce *Mycobacterium tuberculosis,* par l'analyse des séquences desdits fragments de zones appelés MST.

Avantageusement, on détecte une croissance de bactérie du complexe *Mycobacterium tuberculosis* en moins de 15 jours, de préférence en 10 jours au plus.

Les milieux de culture selon l'invention permettent en outre la détection de 50% de l'ensemble des différentes principales espèces de mycobactéries en moins d'une semaine, à savoir *Mycobacterium marinum, Mycobacterium avium,* les bactéries des espèces *Mycobacterium tuberculosis, Mycobacterium bovis* et ses clones ou sous-espèces BCG, *Mycobacterium africanum, Mycobacterium canettii, Mycobacterium caprae, Mycobacterium microti, Mycobacterium pinnipedii Mycobacterium mungi* et *Mycobacterium suricattae.*

Plus particulièrement, ledit prélèvement clinique est choisi parmi des prélèvements respiratoires tels qu'expectoration, liquide de lavage broncho alvéolaire, et des prélèvements de biopsie cutanée, de biopsie ganglionnaire, de biopsie pulmonaire et de biopsie osseuse, et des prélèvements de selles.

Dans un mode préféré de réalisation dans lequel on réalise une culture d'un échantillon de prélèvement clinique pouvant contenir une mycobactérie et des bactéries contaminantes dont la croissance peut inhiber la croissance des mycobactéries, le procédé est caractérisé en ce que :
1/ on réalise une étape préalable de décontamination initiale dudit échantillon dans ledit milieu de culture avec de la chlorhexidine pendant une période limitée, afin de limiter l'action de la chlorhexidine à une activité contre les bactéries autres que mycobactéries, de préférence environ 15 minutes de traitement dans une solution de chlorhexidine à 1% sous agitation, et
2/ on élimine la chlorhexidine en lavant l'échantillon ainsi traité de l'étape 1/ avec un tampon neutre, et on centrifuge et récupère le culot bactérien contenant les bactéries contaminantes ainsi inactivées et les mycobactéries non inactivées, que l'on inocule sur un dit milieu de culture de mycobactéries.

L'utilisation de chlorhexidine comme décontaminant était limitée, précédemment, à la décontamination d'échantillons dans des milieux de culture solides, car la chlorhexidine entraîne une précipitation des milieux de culture liquides. En outre, la chlorhexidine est, en principe, connue pour être toxique sur les mycobactéries. Toutefois, on avait découvert précédemment que la présence de lécithine d'oeuf (mais pas la lécithine de soja) dans le milieu de culture permettait d'éviter ou de différer la précipitation du milieu de culture liquide, d'une part, et, d'autre part, l'action limitée dans le temps de la chlorhexidine, par la mise en oeuvre de ladite étape de lavage 2/, permet d'inhiber l'activité postérieure de la chlorhexidine à l'égard des mycobactéries. La chlorhexidine conserve toutefois son activité première contre les bactéries de la flore commensale dont la croissance peut inhiber la croissance des mycobactéries, appelées ci-dessus bactéries contaminantes.

On procède, en particulier, à cette étape de décontamination préalable pour les prélèvements cliniques connus pour être porteurs d'une flore commensale, tels que des prélèvements d'expectoration, de biopsie cutanée ou de selles, lesquels représentent 95% des prélèvements biologiques disponibles pour une analyse de mycobactéries. En revanche, pour des prélèvements biologiques tels que provenant d'hémoculture ou de biopsie ganglionnaire ou de biopsie pulmonaire ou de biopsie osseuse, une telle décontamination n'est pas requise.

Le procédé ci-dessus est aussi particulièrement avantageux lorsque ledit prélèvement clinique est effectué à partir de selles.

Ledit milieu de culture est un milieu de culture solide et on détecte une croissance de dites mycobactéries à l'oeil nu lorsque l'on peut apercevoir la formation d'une colonie de bactéries sur ledit milieu de culture solide.

En général, l'observation de la formation d'une colonie bactérienne correspondant à une concentration en bactéries de 10⁶ bactéries/ml.

Dans un mode préféré de réalisation d'un procédé selon l'invention, l'on identifie l'espèce de bactéries du genre mycobactérie par des moyens d'analyse moléculaire consistant dans une analyse du profil protéique obtenu par spectrométrie de masse, en le comparant à une série de spectres de profil protéique obtenus avec des échantillons de souche de mycobactéries de référence de différentes espèces cultivées dans les mêmes conditions de culture.

On entend ici par "mêmes conditions de culture" que l'on a recours au même milieu de culture, même température de culture pour une même espèce de mycobactérie.

Un milieu de culture solide selon la présente invention permet de réduire le délai de croissance des colonies de *M. tuberculosis* en présence d'antibiotique et donc obtenir rapidement des résultats d'antibiogramme. Par ailleurs, on a découvert selon la présente invention qu'il était possible d'utiliser avantageusement le procédé E-test pour la réalisation des antibiogrammes de *M. tuberculosis.* Le E-test (AB Biodisk, Solna, Suède ; bioMérieux, Marcy-l'Etoile, France) consiste en une bande de papier imprégné d'un gradient de concentration d'un antibiotique étudié, permettant une lecture directe de la concentration provoquant l'inhibition de croissance de la bactérie (Concentration Minimale Inhibitrice, CMI). Le E-test a été validé pour *M. tuberculosis* sur des milieux de référence différents de celui selon l'invention [Esteban J. et al. Eur J Clin Microbiol Infect Dis 2005 ; 24: 856-857] et les caractéristiques du milieu de culture solide selon l'invention ne permettaient pas prévoir s'il serait approprié à la réalisation des E-tests.

On a démontré selon la présente invention que le milieu de culture solide selon l'invention permet également une réalisation plus rapide et une lecture plus facile d'un test phénotypique de sensibilité et résistance aux antibiotiques du complexe *Mycobacterium tuberculosis* que les milieux standards.

La présente invention permet donc de mettre à profit (Invention d'un milieu permettant l'isolement et la croissance rapide du complexe *Mycobacterium tuberculosis,* pour la réalisation rapide de tests phénotypiques de sensibilité du complexe *Mycobacterium tuberculosis* aux antibiotiques antituberculeux comme illustré dans l'exemple.

Plus précisément, la présente invention fournit un procédé de culture permettant la détermination phénotypique rapide de la sensibilité d'une mycobactérie aux antibiotiques, selon laquelle on réalise les étapes dans lesquelles :
i/- on réalise une culture d'une dite mycobactérie, de préférence du complexe *Mycobacterium tuberculosis,* sur dit un milieu de culture solide en présence d'au moins un antibiotique donné, à différentes concentrations connues, et
ii/- on détermine la plus petite concentration d'antibiotique, de préférence choisi parmi la rifampicine, isoniazide, éthambutol, pyrazinamide et la streptomycine, qui inhibe toute croissance visible de ladite bactérie (CMI : concentration minimale inhibitrice).

Ainsi, de façon connue, pour chaque couple de mycobactérie-antibiotique, on peut déterminer une concentration minimale inhibitrice ou CMI et la comparer aux concentrations critiques d'antibiotiques qu'un malade peut recevoir sans danger et qui bloque la croissance de la souche bactérienne en cause. On détermine alors la sensibilité ou la résistance de la bactérie à l'antibiotique comme suit :
- si la CMI est inférieure à la concentration critique inférieure, la bactérie est sensible à l'antibiotique, et
- si la CMI est supérieure à la concentration critique supérieure, la bactérie est résistante à l'antibiotique en cause.

Dans un mode préféré de réalisation selon l'invention, à l'étape i/-, on dépose, sur ledit milieu de culture solide, au moins une bandelette de papier imprégné d'un dit antibiotique, à différentes concentrations selon un gradient de concentration le long de ladite bandelette.

Plus particulièrement, on ensemence une boite de Pétri comprenant ledit milieu de culture solide selon l'invention à une concentration d'au moins 10⁴ colonies/ml, de préférence 10⁶ colonies/ml, puis on dépose une dite bandelette sur ledit milieu de culture solide, notamment une bandelette de type E-test qui comprend un gradient continu en concentrations croissantes d'antibiotique d'une extrémité à l'autre de ladite bandelette, lesdites concentrations d'antibiotique étant écrites directement sur la bandelette, et on met en incubation la boite sur laquelle repose la bandelette et on lit la CMI à l'intersection du disque d'inhibition de la croissance bactérienne avec la bandelette.

Selon d'autres modes de réalisation connus, on peut remplacer la bandelette par un disque de papier buvard imprégné de la concentration critique d'antibiotique, afin de vérifier l'existence d'une zone d'inhibition autour du disque pour établir que la souche est sensible à l'antibiotique.

Selon une autre variante de réalisation connue, on peut incorporer l'antibiotique directement dans la masse dudit milieu de culture solide à ladite concentration critique et comparer la croissance de la souche sur ledit milieu de culture solide imprégné dudit antibiotique, avec la croissance d'une même souche sur un même milieu de culture solide ne contenant pas d'antibiotique, pour vérifier la présence ou l'absence d'une croissance bactérienne dans le milieu de culture avec et, respectivement, sans antibiotique et en déduire que la souche est sensible à l'antibiotique si l'on observe une croissance moindre de la souche dans le milieu de culture solide contenant ledit antibiotique.

D'autres caractéristiques et avantages de la présente invention ressortiront mieux à la lecture des exemples non limitatifs qui suivent en lien avec les dessins dans lesquelles :
La figure 1 est une représentation graphique du délai de détection t (en heures) de *Mycobacterium tuberculosis* sur le milieu selon l'invention par comparaison avec le milieu Colestos, en utilisant des inocula de différentes concentrations inoculées de 10², 10³, 10⁴ cfu/ml.
La figure 2 est une représentation graphique du délai de détection t (en heures) de *Mycobacterium tuberculosis* sur le milieu selon l'invention par comparaison avec le milieu Löwenstein-Jensen (« L-J »), en utilisant des inocula de différentes concentrations inoculées de 10², 10³, 10⁴ cfu/ml.

### EXEMPLE 1: DETERMINATION DE LA COMPOSITION DU MILIEU SELON L'INVENTION

1) Dans le but d'éliminer le sang présent dans le milieu solide MOD-5% similaire à celui de de l'exemple 2C de EP 2 364 357, les inventeurs - après de nombreuses tentatives infructueuses- ont substitué avec succès au sang du mouton une concentration équivalente (5%) de sérum d'agneau pour définir un milieu MOD-LS (MOD-Lamb Serum). Les performances de ce milieu MOD-LS ont été comparées à celles du milieu MOD et d'un milieu MOD sans sang de mouton MOD4-WB (MOD-without blood).

La composition dudit milieu MOD-5% est donnée ci-dessous pour 1000 mL:
Eau distillée stérile: 841.3 mL
1- Milieu de Middlebrook 7H10 :
   Sulfate d'ammonium : 0,50 g
   Phosphate monopotassique : 1,50 g
   Phosphate disodique : 1,50 g
   Citrate de sodium : 0,4 g
   Sulfate de magnésium : 0,05 g
   Chlorure de calcium : 0,00050 g
   Sulfate de zinc : 0,0010 g
   Sulfate de cuivre : 0,0010 g
   Acide L-glutamique : 0,50 g
   Citrate d'ammonium ferrique : 0,04 g
   Chlorhydrate de pyridoxine : 0,0010 g
   Biotine : 0,00050 g
   Vert malachite : 0,000250 g
   Gélose (Agar): 13.5 g
2- Facteurs de croissance additionnels:
   Albumine bovine : 7.5 g
   Dextrose : 3 g
   Catalase : 0.0045 g
   Acide oléique : 0.09 g
   Bovine serum albumin : 1.65 g
   Nicotinamide Adenine Dinucleotide (NAD) : 0.022 g
   Vinylpyrrolidine Copolymer : 1.67 mL
   Acide citrique : 0.0842 g
   Hemin 330 µg 0.00033 g
   Tween 80 : 2 mL
   Extrait de levure : 1g
   Pancreatic digest of casein: 1g
   Glucose: 2g
   Glycérol: 5 mL
3- Antibiotiques sans activité antimycobactérienne et antifungique :
   Polymyxine : 20.000 unités
   Amphothéricine B : 0.002 g
   Acide nalidixique : 0.008 g
   Triméthoprime : 0.002 g
   Azlocilline : 0.002 g
   Vancomycine : 0.005 g
4- Eléments additionnels:
   Sérum de veau foetal: 50 ml
   Sang de mouton défibriné : 50 ml
   Lécithine : 5 g

Pour MOD-LS-5%, la composition est identique pour les composants des points 1 à 3 et présente les dits éléments additionnels suivants:
4- Eléments additionnels:
Sérum d'agneau: 50 ml
Lécithine : 5 g.

Pour MOD-WS-5%, la composition est identique pour les composants des points 1 à 3 et présente les dits éléments additionnels suivants:
4- Eléments additionnels:
Sérum de veau foetal: 50 ml
Lécithine : 5 g

Cinq souches de *Mycobacterium tuberculosis* ont été utilisées, la souche de référence *Mycobacterium tuberculosis* H37Rv et quatre souches cliniques dont une souche de la famille Beijing. Des suspensions de concentration égale à 10⁵ CFU/mL ont été préparées à partir de ces souches et 100 µL de chaque suspension ont été inoculés en triplicate sur les trois milieux.

Les milieux ont été incubés en parallèle à 37°C sous une atmosphère contenant 5% de CO2 et sous micro aérophile (tension oxygène entre 2,5% et 5%).

Le délai moyen d'apparition des premières colonies était 4 ± 0,3 jour sur MOD, 3.8 ± 0,4 jours sur MOD-LS et 5.1 ± 0,6 jours sur MOD-WS. Il y avait pas de différence significative quant au délai d'apparition des colonies sur milieu MOD comparé au milieu MOD-LS (P = 0.1413). En revanche, en comparant le milieu MOD-WS au milieu MOD et au milieu MOD-LS, la différence était plus significative (P < 0,05). Ces résultats indiquent qu'il est avantageux de substituer du sérum d'agneau, au sang pour obtenir des résultats de croissance légèrement améliorés.

Dans un deuxième temps, les inventeurs ont optimisé la concentration et le type de sérum d'agneau à introduire dans le milieu. Une variété de milieux a été préparée:
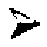 MOD-15%-HIBS idem à MOD-WB-5% mais avec 15% (150mL) sérum bovin décomplémenté au lieu de 5% de sérum bovin non décomplémenté
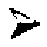 MOD-15%-B5: 15%(150mL) sérum bovin non décomplementé
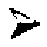 MOD-15%-HILS: 15% (150mL) sérum d'agneau décomplementé
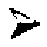 MOD-15%-LS: 15% (150mL) sérum d'agneau non décomplementé
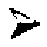 MOD-10%-LS: 10%(100mL) sérum d'agneau non décomplementé
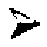 MOD-5%-L5: 5% (50mL) sérum d'agneau non décomplementé
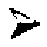 MOD-15%-HIBS-5%-LS: 15% (150mL) sérum bovin décomplementé et 5% (50mL) sérum d'agneau non décomplementé
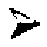 MOD-15%-HIBS-5%-HILS: 15% (150mL) sérum bovin décomplementé et 5% (50mL) sérum d'agneau décomplementé
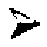 MOD-15%-BS-5%-LS: 15% (150mL) sérum bovin non décomplementé et 5% (50mL) sérum d'agneau non décomplementé

Les performances de ces milieux ont été mesurées en cultivant cinq souches de *Mycobacterium tuberculosis* dont la souche de référence *Mycobacterium tuberculosis* H37Rv et quatre souches cliniques dont une souche de la famille Beijing. Des suspensions de concentration égale à 105 CFU/mL ont été préparées à partir de ces souches et 100 µL de chaque suspension ont été inoculés en triplicata sur les différents milieux.

Deux critères ont été pris comme base pour la comparaison des performances de ces différents milieux de culture:
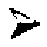 Délai de détection des premières colonies,
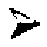 Densité et richesse des colonies après 7 jours d'incubation. Suite à une inspection visuelle, un gradient de 1 (plus faible) à 5 (plus fort) est donné à chaque boite de Pétri pour mesurer la richesse de la culture.

Les résultats sont reproduits dans le tableau 1 ci-dessous (en boîtes de Pétri).

**Tableau 1**

| | MOD-15%-HIBS | MOD-15%-BS | MOD-15%-HILS | MOD-15%-LS | MOD-10%-LS | MOD-5%-LS | MOD-15%-HIBS-5%-LS | MOD-15%-HIBS-5%-HILS | MOD-15%-BS-5%-LS |
|---|---|---|---|---|---|---|---|---|---|
| Délais de détection (heures) | 101 ± 10.8 | 102 ± 12 | 87 ± 13.6 | 88 ± 14.7 | 91 ± 14.8 | 98 ± 10 | 89 ± 13 | 90 ± 17 | 95 ± 12 |
| Densité / Richesse | 2 ± 0.8 | 2.2 ± 0.7 | 3.5 ± 1.4 | 2.8 ± 1.1 | 2.3 ± 0.8 | 2 ± 0.8 | 3 ± 1.1 | 3 ± 1.3 | 3 ± 1.3 |

En comparant les milieux contenant 15% de sérum bovin et 5% de sérum d'agneau décomplémenté ou non décomplémenté (MOD-15%-HIBS-5%-HILS, MOD-15%HIBS-5%-LS et MOD-15%-BS-5%-LS), aucune différence significative n'est observée quant au délai de détection des colonies ou la richesse des colonies.

Par ailleurs, on n'observe pas d'effet significatif de la décomplémentation du sérum bovin (MOD-15%-HIBS versus MOD-15%-BS) sur le délai de détection (P = 0,8321), ni sur la richesse des colonies (P = 0,6030).

Il n'existe pas non plus d'effet significatif de la décomplémentation du sérum d'agneau (MOD-15%-HILS versus MOD-15%-LS) sur le délai de détection (P = 0,8649) des colonies. En revanche, on observe un score de richesse le plus élevé sur milieu MOD-15%-HILS.

On observe un délai de détection des colonies significativement inférieur sur milieux incorporant du sérum d'agneau (MOD-15%-HILS et MOD-15%-LS) à celui observé sur milieux incorporant du sérum bovin (P < 0.05).

Plus particulièrement, le milieu MOD-15%-HILS présente un score de densité et de richesse significativement supérieure à ceux présentés par les milieux à base de sérum bovin.

Il n'y a pas de différence statistiquement significative quant au délai de détection des colonies ou au score de richesse des colonies en comparant le milieu MOD-15%-HILS et le MOD-15%HIBS-5%HILS.

En diminuant progressivement la concentration du sérum d'agneau (MOD-15°/o-LS, MOD-10%-LS et MOD-5%-LS), on observe une augmentation progressive du délai de détection et une diminution progressive du score de densité et de richesse des colonies.

Les colonies ont été détectées en parallèle par oeil nu ou par auto fluorescence utilisant une binoculaire équipée de fluorescence et une caméra monochrome (MZ10F stéréomicroscope, Leica Microsystèmes SAS, Nanterre, France) équipée d'un filtre GF1.

A partir de ces résultats, les inventeurs ont décidé d'utiliser le milieu MOD-15%-HILS pour la suite des travaux de mise au point de la capacité de détection visuelle des colonies à l'oeil nu.
2) Les inventeurs ont ensuite travaillé à contraster la couleur blanc-beige des colonies de *Mycobacterium tuberculosis* sur le fond jaune des milieux de culture MOD ci-dessus pour augmenter la capacité de détection visuelle à l'oeil nu de la croissance des mycobactéries.

Différents lots de milieu MOD-15%-HILS ont été préparés et colorés en utilisant différents colorants neutres de différentes couleurs et à différentes concentrations.

Pour ce faire, les inventeurs ont utilisé dans un premier temps des colorants alimentaires pour leur innocuité et leur disponibilité afin d'apprécier lequel ou lesquels de ces colorants permettraient d'obtenir un contraste satisfaisant entre les colonies de mycobactéries et le fond de la gélose. Les colorants alimentaires suivants ont été incorporés dans le milieu selon l'invention pour obtenir une gamme de couleurs.
Bleu-ciel : 0.4% colorant bleu
Bleu-pale : 0.2% colorant bleu
Jaune : 0.4% colorant jaune
Saumon : 0.04% colorant rouge
Orange : 0.3% colorant jaune + 0.1% colorant rouge
Orange-rougeatre : 0.2% colorant rouge + 0.2% colorant jaune
Rouge clair : 0.3% colorant rouge + 0.1% colorant jaune
Rouge : 0.4% colorant rouge (Couleur choisie pour MOD9)
Rouge-bordeaux : 0.3% colorant rouge + 0.1% colorant bleu
Violet-rougeatre : 0.2% colorant rouge + 0.2% colorant bleu
Violet : 0.3% colorant rouge + 0.1% colorant jaune
Vert-jaunatre : 0.3% colorant jaune + 0.1% colorant bleu
Vert-pistache : 0.2% colorant jaune + 0.2% colorant bleu
Vert : 0.3% colorant bleu + 0.1% colorant jaune
Maron : 0.2% colorant bleu + 0.1% colorant jaune + 0.1% colorant rouge

Finalement, c'est la couleur rouge qui a été retenue. Les inventeurs ont alors déterminé que ce colorant alimentaire contenait de l'azorubine et du Rouge Ponceau.

L'azorubine ou carmoisine est un colorant alimentaire rouge identifié sous le numéro E122. Ce composé azoté est un sel disodique de l'acide hydroxy-1 (sulfo-4 naphtylazo)-2 naphtalènesulfonique-4.

Le rouge de Ponceau est un colorant azoïque de synthèse (C₂₀H₁₁N₂Na₃O₁₁S₃) dénommé aussi C.I. 16255, Rouge cochenille A, utilisé comme colorant alimentaire (E124² aussi dénommé additif E124.

La combinaison d'azorubine 50mg/L et de Ponceau 4R 30mg/L colorant en rouge le milieu MOD-15%-HILS a présenté le meilleur contraste colonie/gélose.

Le milieu MOD-15%-HILS dans lequel le sang de mouton (5%) et le sérum bovin décomplémenté (15%) ont été substitués par le sérum d'agneau décomplémenté (15%) et le mélange d'azorubine (50mg/L) et Ponceau 4R (30mg/L) est appelé "MOD9".

La composition dudit milieu MOD9 selon l'invention est donnée ci-dessous :
Milieu solide MOD9 pour 1000 mL:
Eau distillée stérile: 841.3 mL
1- Milieu de Middlebrook 7H10 :
   Sulfate d'ammonium : 0,50 g
   Phosphate monopotassique : 1,50 g
   Phosphate disodique : 1,50 g
   Citrate de sodium : 0,4 g
   Sulfate de magnésium : 0,05 g
   Chlorure de calcium : 0,00050 g
   Sulfate de zinc : 0,0010 g
   Sulfate de cuivre : 0,0010 g
   Acide L-glutamique : 0,50 g
   Citrate d'ammonium ferrique : 0,04 g
   Chlorhydrate de pyridoxine : 0,0010 g
   Biotine : 0,00050 g
   Vert malachite : 0,000250 g
   Gélose (Agar): 13.5 g
2- Facteurs de croissance additionnels:
   Albumine bovine : 7.5 g
   Dextrose : 3 g
   Catalase : 0.0045 g
   Acide oléique : 0.09 g
   Bovine serum albumin : 1.65 g
   Nicotinamide Adenine Dinucleotide (NAD) : 0.022g
   Vinylpyrrolidine Copolymer : 1.67 mL
   Acide citrique : 0.0842 g
   Hemin 0.00033 gTween 80 : 2 mL
   Extrait de levure : 1g
   Pancreatic digest of casein: 1g
   Glucose: 2g
   Glycérol: 5 mL
3- Antibiotiques sans activité antimycobactérienne et antifungique :
   Polymyxine : 20.000 unités
   Amphothéricine B : 0.002 g
   Acide nalidixique : 0.008 g
   Triméthoprime : 0.002 g
   Azlocilline : 0.002g
   Vancomycine : 0.005g
4- Eléments additionnels de l'invention :
   Sérum d'agneau décomplémenté: 150 mL
   Lécithine : 5 g
   Azorubine : 0.050 g
   Ponceau 4R : 0.030 g

Dans une variante de réalisation, on a ajouté 0.1g d'acide ascorbique ce qui a permis de s'affranchir du contrôle de la tension en oxygène inférieure à 5% et de réaliser une culture en atmopshère de 5% de CO2 voire à l'air libre et atmosphère ambiante à la même température de 37°C.

### Exemple 2 : INFLUENCE DE LA COULEUR ROUGE DU MILIEU MOD9 SUR LA CROISSANCE DE M. TUBERCULOSIS A L'AIDE DE COLORANTS CHIMIQUEMENT DEFINIS.

Dans cet exemple, les inventeurs ont comparé la couleur rouge du milieu MOD9 obtenue par addition d'azorubine seule, de rouge Ponceau seul, ou d'un mélange des deux colorants ; et l'Influence de ces trois formulations sur la croissance de *M. tuberculosis*

### a) Milieux:

- MOD9-A: Le colorant est de l'azorubine 50mg/L
- MOD9-P: Le colorant est du Ponceau 4R 30mg/L
- MOD9-A/P: Le colorant est un mélange d'azorubine 50mg/L et de rouge Ponceau 4R (30mg/L)

Deux expérimentations ont été menées :
- Sur les différents milieux coulés en boite de Pétri (P-MOD9)
- Sur les différents milieux coulés en tubes (bioMérieux) (T-MOD9)

### b) Souches:

Les tests effectués sur les milieux en format boite de Pétri sont réalisés avec cinq souches de *Mycobacterium tuberculosis,* la souche de référence H37Rv et 4 souches cliniques, sensibles aux antituberculeux dont une est Beijing ; alors que les tests effectués sur les milieux en format tube sont réalisés avec deux souches de *M. tuberculosis,* la souche de référence H37Rv et une souche Beijing.

### c) Protocole:

Les souches maintenues en culture sur milieu MOD9 sont calibrées à 10⁶ cfu/mL par observation microscopique après coloration de Ziehl-Neelsen.

De plus des suspensions plus diluées, 10⁴ cfu/mL et 10² cfu/mL sont préparées.

Les expérimentations sont réalisées en triplicata pour les tests lancés sur format boite de Pétri et en duplicata sur format tubes (Limité par rapport au nombre des tubes).

Les suspensions préparées sont inoculées à raison de 100 µL pour les boites de Pétri et 200 µL pour les tubes et incubées dans un incubateur 37°C, 5% CO2 (avec les bouchons semi-ouverts pour le format tube).

Après inoculation, les tubes sont mis en position horizontale pendant 3 jours, puis redressés en position verticale jusqu'à la fin du test.

Des milieux non ensemencés utilisés comme témoins négatifs sont incubés en parallèle.

Une lecture à l'oeil nu des milieux est réalisée toutes les 12 heures pour la détection des colonies. L'identification des colonies est confirmée ultérieurement par coloration Ziehl-Neelsen.

### d) Résultats

- Couleur : les inventeurs ont observé une différence de l'intensité de la couleur rouge du milieu de culture MOD9, avec une intensité croissante en allant du milieu coloré par la rouge Ponceau seul, l'azorubine seule, et le mélange rouge Ponceau plus azorubine.
- Croissance de *M. tuberculosis* : Les résultats (moyenne et écart type) du délai de détection de la première colonie des différentes souches de *M. tuberculosis* sur les différents milieux est présentés dans les tableaux 2 et 3 ci-dessous.

**Tableau 2 : Temps de détection de la première colonie des différentes souches de M. tuberculosis sur les différents milieux en boite de Pétri**

| Délai de détection heures | 10⁶ CFU/mL | 10⁴ CFU/mL | 10² CFU/mL |
|---|---|---|---|
| P-MOD9-A/P | 32 ± 8.7 | 82.4 ± 20.2 | 125.6 ± 21.2 |
| P-MOD9-A | 32.8 ± 10.6 | 79.2 ± 22.1 | 124.8 ± 23.9 |
| P-MOD9-P | 33.6 ± 11.3 | 81.6 ± 20.8 | 136 ± 19.6 |

**Tableau 3 : Temps de détection de la première colonie des différentes souches de M. tuberculosis sur les différents milieux en tube**

| Délai de détection (heures) | 10⁶ CFU/mL | 10⁴ CFU/mL | 10² CFU/mL |
|---|---|---|---|
| T-MOD9-A/P | 51 ± 18 | 105 ± 15.1 | 162 ± 37.3 |
| T-MOD9-A | 63 ± 11.5 | 114 ± 12 | 171 ± 24.7 |
| T-MOD9-P | 60 ± 9.8 | 107.2 ± 9.9 | 186 ± 20.8 |

Les inventeurs n'ont pas observé de différence significative quant au délai de détection entre les différents colorants et combinaisons utilisés pour colorer le milieu MOD9 en rouge.

Au total, l'ensemble des résultats indique qu'il convient de colorer le milieu MOD9 en rouge par un mélange azorubine à 50 mg/L plus rouge Ponceau à 30 mg/L.

### Exemple 3 : STABILITE DE LA COULEUR ROUGE DU MILIEU MOD9

Dans cet exemple, les inventeurs se sont attachés à vérifier la stabilité dans le temps de la couleur rouge framboise du milieu MOD9 sur gélose, appréciée par son apparence à l'oeil dans les conditions habituelles de stockage à 4°C et d'utilisation.

Pour cela, les inventeurs ont préparé un premier lot 6 géloses MOD9 en boîte de Pétri de 55 mm, laissées dans un chambre froide à température contrôlée à 4°C correspondant à la température habituelle de stockage des milieux contenant des composés organiques. La couleur des boîtes a été observée le jour J0, puis à J7, J14, J21, J30, J45 puis deux mois, trois mois et six mois après incubation. Une photographie numérique du groupe de gélose a été enregistrée à chaque observation. Dans ces conditions, les inventeurs n'ont pas observé de modification de la couleur rouge du milieu MOD9 dans aucune des conditions expérimentales, indiquant la grande stabilité de coloration du milieu MOD9 dans la composition indiquée dans l'exemple 1, conservé à 4°C.

Un deuxième lot de 6 boîtes de Pétri contenant du MOD9 a été incubé en air ambiant à température du laboratoire comprise entre 18°C et 22°C pendant 45 jours et observé dans les mêmes conditions. Dans ces conditions, les inventeurs n'ont observé aucune modification de la couleur rouge, indiquant la possibilité d'un stockage dans l'ambiance d'un laboratoire, plus particulièrement dans des laboratoires ne disposant pas de ressources suffisantes en froid.

Un troisième lot de 6 boîtes de Pétri contenant du MOD9 a été incubé à 37°C dans un incubateur contenant 5% de CO2 pendant 45 jours et observé dans les mêmes conditions. Dans ces conditions, les inventeurs n'ont observé aucune modification de la couleur rouge, indiquant la possibilité d'incuber le milieu MOD9 en suivant les recommandations internationales pour la détection des colonies de mycobactéries, plus particulièrement des colonies de *Mycobacterium tuberculosis.*

En parallèle, les inventeurs ont mené une deuxième série d'expériences dans un deuxième laboratoire aux mêmes températures et pendant 5 semaines au cours desquelles ils ont observé une même coloration rouge framboise et la stérilité du milieu MOD9. Ces résultats sont concordants avec ceux menés dans le premier laboratoire.

### Exemple 4: PERFORMANCES COMPAREES DU MILIEU MOD9 ET DU MILIEU COLETSOS POUR LA CROISSANCE DES SOUCHES DE M. TUBERCULOSIS

Dans cet exemple, les inventeurs ont comparé la performance du milieu MOD9 dans des boites de Pétri 55 mm à celles du milieu Coletsos en tubes de verre. Le milieu Coletsos est un milieu à l'oeuf comprenant du pyruvate de sodium.

On a utilisé des suspensions de 15 souches de *Mycobacterium tuberculosis* préparées à différentes concentrations. Les 15 souches de *Mycobacterium tuberculosis* utilisées comprennent la souche de référence *Mycobacterium tuberculosis* H37Rv et 14 isolats cliniques dont deux isolats de la famille Beijing.

Des suspensions de 10⁴, 10³ et 10² CFU/mL ont été préparées à partir de ces souches. Les 2 milieux ont été inoculés en triplicata avec 100µL de chaque suspension, puis incubés à 37°C dans une atmosphère contenant 5% CO2, la lecture des résultats était faite toutes les 12h afin de déterminer le délai de détection des premières colonies de *M*. *tuberculosis.*

Les délais de détection des premières colonies sur chacun des milieux sont repris dans le tableau 4 ci-dessous :

**TABLEAU 4**

| | Délai de détection (jours) | |
|---|---|---|
| | MOD9 (Boite de Pétri) | Coletsos (Tube) |
| 10⁴ CFU/mL | 2.5 ± 0.4 * | 6.46 ± 1.1 |
| 10³ CFU/mL | 3.4 ± 0.37 * | 7.96 ± 1 |
| 10² CFU/mL | 4.2 ± 0.87 * | 9.2 ± 1.87 |

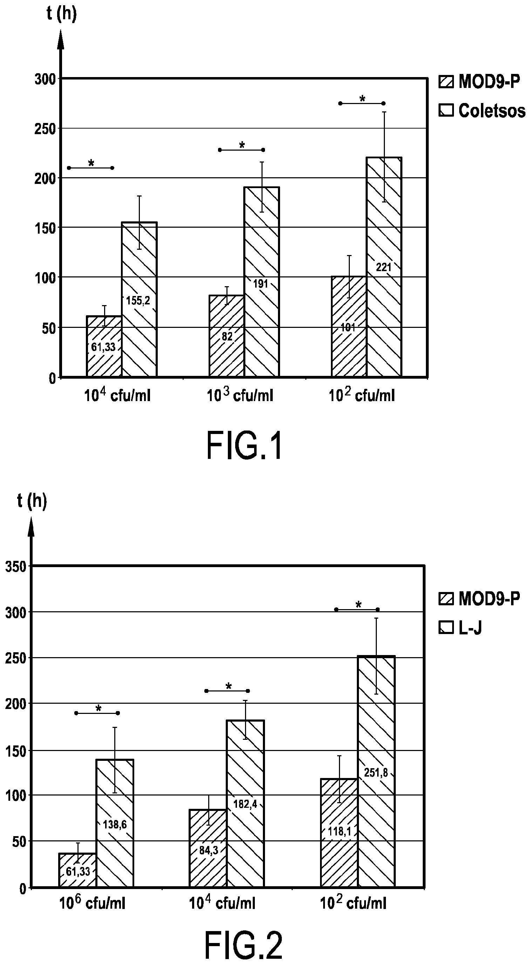

On observe un délai de détection des colonies significativement inférieur sur milieu MOD9en boîtes de Pétri par rapport au milieu Coletsos en tubes, quelle que soit la concentration testée. Ces résultats sont illustrés dans la Figure 1.

### Exemple 5 : PERFORMANCES COMPAREES DU MILIEU MOD9 ET DU MILIEU LOWENSTEIN-JENSEN POUR LA CROISSANCE DES SOUCHES DE M. TUBERCULOSIS

Les performances du milieu MOD9 en boite de Pétri 55 mm ont été comparées à celles du milieu Löwenstein-Jensen (bioMérieux, La Balme-les-Grottes, France) en tubes en verre. Un total de 21 souches de *Mycobacterium tuberculosis* ont été utilisées, comprenant la souche de référence *Mycobacterium tuberculosis* H37Rv et 20 isolats cliniques dont deux appartenant à la famille Beijing. Des suspensions de 10⁶, 10⁴ et 10² CFU/mL ont été préparées à partir de ces souches. Les 2 milieux ont été inoculés en triplicata avec 100µL de chaque suspension, puis incubés à 37°C dans une atmosphère contenant 5% CO2. L'observation des milieux de culture est faite toutes les 12 h afin de déterminer le délai de détection des premières colonies de *Mycobacterium tuberculosis.*

Les délais de détection des premières colonies sur chacun des milieux sont repris dans le tableau 5 ci-dessous :

**TABLEAU 5**

| | Délai de détection (jours) | |
|---|---|---|
| | MOD9 (Boite de Pétri) | Löwenstein-Jensen (Tube) |
| 10⁶ CFU/mL | 1.5 ± 0.4 * | 5.7 ± 1.5 |
| 10⁴ CFU/mL | 3.5 ± 0.6 * | 7.6 ± 0.8 |
| 10² CFU/mL | 4.9 ± 1 * | 10.8 ± 1.7 |

On observe un délai de détection des colonies significativement inférieur sur milieu MOD9 en boîtes de Pétri par rapport au milieu Lowenstein-Jensen en tube, quelle que soit la concentration testée. Ces résultats sont illustrés dans la Fgure 2.

### Exemple 6: PERFORMANCES COMPAREES DU MILIEU MOD9 ET DU MILIEU DE LOWENSTEIN-JENSEN POUR L'ISOLEMENT DES SOUCHES DE MYCOBACTERIES

Dans cet exemple, les inventeurs ont observé les performances du milieu MOD9 dans l'isolement des mycobactéries des échantillons cliniques. Un total de 250 échantillons dont 203 (81.2%) échantillons respiratoires [(173 crachats (69.2%), 27 aspirations bronchiques (10.8%) et trois lavages broncho-alvéolaires (1.2%)] et 47 (18.8%) échantillons non-respiratoires [36 selles (14.4%) et 11 urines (4.4%)] collectés chez 145 patients ont été décontaminés en utilisant la chlorhexidine (0.7%) (Asmar, 2014) et puis cultivés (100µL d'inoculum) en parallèle sur MOD9 (boite de Pétri 55 mm) et tubes Lowenstein-Jensen (bioMérieux, FRANCE).

Un total 40 mycobactéries ont été isolées à partir des échantillons collectés chez 13 patients différents, Ces isolats ont été identifiés comme 38 *M. tuberculosis* et 2 *M*. *abscessus.* Le nombre d'isolats obtenus sur MOD9 [38 isolats (36 *M. tuberculosis* et 2 *M. abscessus*)] à partir de 26 échantillons de crachats, 11 échantillons de selles et un échantillon d'aspiration bronchique était supérieur aux 32 isolats obtenus sur milieu Lowenstein-Jensen (30 *M. tuberculosis* et 2 *M. abscessus*) à partir de 27 crachats et 5 échantillons de selles (cette différence est significative, Test statistique de Fisher avec une valeur P < 0.5195). Au total, 8 isolats de *M. tuberculosis* ont été obtenus seulement sur milieu MOD9 et 2 isolats de *M. tuberculosis* ont été obtenus seulement sur milieu Löwenstein-Jensen.

Le délai moyen de détection est de 9.9 ± 4 (4-16) jours pour MOD9 versus 17.5 ± 6.6 (8-35) jours pour Lowenstein-Jensen (P < 0.05). Le délai moyen de détection des colonies de *Mycobacterium tuberculosis* est de 10.3 ± 3.9 (5-18) jours sur MOD9 versus 18.1 ± 6.4 (10-35) jours sur Lowenstein-Jensen (P < 0.05). Le délai moyen de détection des colonies Isolées sur les deux milieux est de 9.6 ± 4 (4-18) jours sur MOD9 versus 16.8 ± 6.2 (8-35) jours sur Lowenstein-Jensen (P < 0.05). Le délai moyen de détection des colonies de *Mycobacterium tuberculosis* isolées sur les deux milieux est 9.5 ± 3.9 (5-18) jours sur MOD9 versus 17.4 ± 5.9 (10-35) jours sur Lowenstein-Jensen (P < 0.05). Le taux de contamination sur MOD9 est 1.6% (3 selles et 1 crachat) et de 4.4% (8 selles, 2 crachats et 1 aspiration bronchique) sur Lowenstein-Jensen (P = 0.1129).

### Exemple 7: PERFORMANCES COMPAREES DU MILIEU MOD9 ET DU MILIEU BACTEC™ POUR L'ISOLEMENT DES SOUCHES DE MYCOBACTERIES

Les inventeurs ont comparé un protocole combinant une décontamination des échantillons cliniques contaminés, par chlorhexidine (0.7%) et ensemencement du milieu MOD9 versus le protocole de référence combinant la décontamination des échantillons cliniques contaminés par NALC-NaOH et ensemencement en milieu liquide BACTEC™-MGIT™ (Becton Dickinson) pour l'isolement des mycobactéries à partir d'échantillons cliniques.

Au total, 300 échantillons comprenant 238 (79.3%) échantillons respiratoires [(203 crachats (67.6%), 31 aspirations bronchiques (10.3%) et 4 lavages broncho-alvéolaires (1.3%)] et 62 (20.6%) échantillons non-respiratoires [43 selles (14.3%), 14 urines (4.6%) et 5 ganglions] collectés chez 161 patients ont été utilisés dans cette étude. Toutes les échantillons (sauf les ganglions) ont été décontaminés en utilisant la méthode de référence NALC-NaOH et puis mis en culture (500 µL d'inoculum) dans un flacon MGIT™ (Becton Dickinson) après ajout de 800 µL du supplément OADC et 500 µL de mélange d'antibiotiques PANTA, puis incubés dans l'automate BACTEC™ (Becton Dickinson). Une quantité comprise entre 0,1 mL et 1 mL de matière biologique restante (selon la disponibilité) ont été décontaminés par la chlorhexidine (0.7%) (Asmar, 2014) puis inoculés (100µL d'inoculum) sur milieu MOD9 (boite de Pétri de 55 mm). Les ganglions ont été homogénéisés et mis en culture directement sans décontamination sur MOD9 et MGIT™.

Au total, 46 mycobactéries ont été isolées à partir des échantillons cliniques collectés chez 17 patients différents, ces souches ont été identifiées comme 44 *Mycobacterium tuberculosis* et 2 *Mycobacterium abscessus.* Le nombre d'isolats obtenus sur MOD9 [43 isolats (41 *M. tuberculosis* et 2 *M. abscessus*)] à partir de 29 échantillons de crachats, 11 échantillons de selles, 1 échantillon d'aspiration bronchique et 3 ganglions, était significativement supérieur aux 31 isolats obtenus sur MGIT™ (30 *M. tuberculosis* et 1 *M. abscessus*) à partir de 23 crachats, 3 selles, 1 aspiration bronchique et 5 ganglions (P < 0.05). Au total, 15 isolats (14 *M. tuberculosis* et 1 *M. abscessus*) étaient obtenus seulement sur MOD9 versus 3 souches de *M. tuberculosis* obtenues uniquement sur MGIT™.

Le délai moyen de détection est de 9.8 ± 3.8 (4-18) jours pour MOD9 versus 14 ± 8 (3-32) jours pour MGIT™ (P < 0.05). Le délai moyen de détection des colonies de *Mycobacterium tuberculosis* est 10 ± 3.7 (5-18) jours sur MOD9 versus 14.2 ± 8 (4-32) jours sur MGIT™ (P < 0.05). Le délai moyen de détection des colonies qui ont cultivé sur les deux milieux est de 8.7 ± 3.5 (4-18) jours sur MOD9 versus 13.3 ± 7 (4-25) jours sur MGIT™ (P < 0.05). Le délai moyen de détection des colonies de *Mycobacterium tuberculosis* qui ont cultivé sur les deux milieux est de 8.9 ± 3.5 (5-18) jours sur MOD9 versus 13.6 ± 7 (4-25) jours sur MGIT™ (P < 0.05). Le taux de contamination observé par le protocole chlorhexidine-0.7%/MOD9 était de 1.33% (3 selles et 1 crachat) et significativement inférieur à celui observé par NALC-NaOH/MGIT™ (4.67% ; 7 selles, 6 crachats et 1 aspiration bronchique) (P < 0.05).

### EXEMPLE 8 : DETERMINATION PHENOTYPIQUE DE LA SENSIBILITE DE M. TUBERCULOSIS AUX ANTIBIOTIQUES

### a/- Méthodes :

Les inventeurs ont testé six souches cliniques de l'espèce *Mycobacterium tuberculosis* isolées de prélèvements respiratoires au cours du diagnostic de routine dans leur laboratoire de microbiologie clinique. Ces six souches ont été identifiées de l'espèce *Mycobacterium tuberculosis* par leur morphologie caractéristique en cordons après coloration de Ziehl, puis par amplification PCR et séquençage de spacers intergéniques [Djelouadji Z et al. A Single-Step Sequencing Method for the Identification of Mycobacterium tuberculosis Complex Species. PLoS Negl Trop Dis. 2008 Jun 18;2(6):e253].

Ces six souches ont été testées sensibles aux antibiotiques antituberculeux de première ligne en utilisant un test phénotypique de référence en milieu solide ne testant que les concentrations dites critiques pour chacun des quatre antibiotiques testés : isoniazide, 0,2 µg/ml ; rifampicine, 1 µg/ml ; ethambutol, 5 µg/ml et streptomycine, 5 µg/ml. [Woods GL, Warren NG, Inderlind CB. Susceptibility test methods : Mycobacteria, Nocardia, and other Actinomycetes in: Manual of Clinical Microbiology, 9ème édition (Nurray PR, Baren EJ, Jorgensen JH, Bry NL, Pfaller MA. American Society for Microbiology, 2007, page 1223-1247].

On a testé en parallèle une souche clinique résistante aux antituberculeux (concentration minimale inhibitrice de la rifampicine > 1 µg/ml) comme déterminé par la méthode de référence en milieu solide [Woods GL, Warren NG, Inderlind CB. Susceptlbility test methods : Mycobacteria, Nocardia, and over actinomycetes in: Manual of Clinical Microbiology, 9ème édition (Nurray PR, Baren EJ, Jorgensen JH, Bry NL, Pfaller MA. American Society for Microbiology, 2007, page 1223-1247). Ces souches ont été mises en suspension dans du tampon phosphate stérile à la concentration de 10⁶ colonies/ml.

Cette suspension a servi à inonder en parallèle une boite de Pétri stérile contenant le milieu standard de référence : Middlebrook 7H10 complémenté par un mélange d'acide oléique, albumine, dextrose, et catalase (OADC), une boîte de Pétri stérile contenant le milieu MOD-15%HIBS et une boîte de Pétri stérile contenant le milieu solide MOD9 selon l'invention de l'exemple 1 ci-dessus.

Les inventeurs ont utilisé le système appelé E-test® (Biomérieux, France) pour déterminer la sensibilité des souches aux différents antibiotiques. Ce système est composé d'une bandelette en papier imprégnée d'antibiotique (isoniazide ou éthambutol) selon un gradient de concentration continu au long de la bandelette. Ce dispositif permet une lecture à l'oeil nu de la concentration à partir de laquelle il n'est plus observé de croissance de *Mycobacterium tuberculosis* définissant ainsi de la concentration minimum inhibitrice de l'antibiotique (isoniazide ou éthambutol) vis-à-vis de la souche de *Mycobacterium tuberculosis* qui est testée. Les boîtes de Pétri ont été incubées à 37°C sous une atmosphère contenant 5% de CO2 pendant deux semaines avec une observation quotidienne à partir du sixième jour d'incubation. Les colonies ont été confirmées comme étant *Mycobacterium tuberculosis* sur la base de leur aspect et après coloration de Ziehl montrant des cordons de bacilles alcoolo-acido-résistants. Il se forme un disque d'inhibition de la croissance bactérienne autour de la bandelette dans les zones contenant une concentration d'antibiotique supérieure à la concentration minimum inhibitrice, étant entendu que celle-ci est déterminée par l'intersection du disque d'inhibition avec la bandelette.

### b/- Résultats :

Les inventeurs ont observé que :
- (1) les colonies de *Mycobacterium tuberculosis* apparaissaient après 7 jours d'incubation sur le milieu MOD9 selon l'invention comme sur le milieu comparatif MOD-15%HIBS.
- (2) la lecture du E-test est possible dans ces conditions après 7 jours d'incubation sur le milieu selon l'invention car il y a un nombre suffisant de colonies permettant la lecture et un très bon contraste entre les colonies qui apparaissent grises sur le fond rouge du milieu MOD 9 selon l'invention; comme sur le milieu MOD-15% HIBS et meilleur que sur le milieu de référence, du fait de la croissance incomplète des colonies sur le milieu de référence et d'un mauvais contraste entre la couleur de la colonie (translucide) et le milieu de référence,
- (3) les résultats des E-tests isoniazide et éthambutol obtenus sur le milieu MOD9 selon l'invention sont similaires à ceux obtenus au préalable avec MOD-15%HIBS-et sont plus précis que ceux des tests de référence, permettant la détermination d'une véritable concentration minimale inhibitrice.

Cette expérience démontre que, par utilisation d'un milieu solide selon l'invention, la réalisation de tests phénotypiques de sensibilité de *Mycobacterium tuberculosis* aux antituberculeux selon la méthode E-test, est aussi rapide et plus facile que la réalisation de E-test sur un milieu solide de référence.

Une boîte de Pétri contenant le milieu de référence et le milieu selon l'invention incubée après inoculation d'une même souche de l'espèce *Mycobacterium tuberculosis,* montre la présence de colonies de *Mycobaderium tuberculosis* facilement visibles après 7 jours sur le milieu selon l'invention et difficilement visibles sur le milieu de référence après 11 jours, permettant une lecture facile de la concentration inhibitrice de isoniazide (IZ) sur une première bandelette et éthambutol (ET) sur une deuxième bandelette.

On voit, autour des bandelettes, des zones d'inhibition de la croissance bactérienne en forme de disque ovoïde autour des parties de la bandelette contenant une concentration inhibitrice d'antibiotique, de sorte que l'intersection de la limite inférieure desdites zones inhibitrices avec les bandelettes permet de lire sur la bandelette graduée la concentration inhibitrice minimale de l'antibiotique.

## Revendications

1. Milieu de culture de mycobactéries apte à permettre la culture de mycobactéries à partir d'un échantillon de prélèvement clinique, comprenant de la lécithine, des facteurs de croissance de mycobactéries et, de préférence, des antibiotiques sans activité à l'égard des mycobactéries, **caractérisé en ce qu'**il ne comprend pas de sang et comprend en outre au moins les composants additionnels suivants :
- du sérum d'agneau, de préférence du sérum d'agneau décomplémenté, et
- au moins un colorant rouge sans activité contre les mycobactéries, permettant de contraster la couleur du milieu de culture par rapport aux colonies de mycobactéries.

2. Milieu de culture de mycobactéries selon la revendication 1, **caractérisé en ce que** le sérum d'agneau est compris dans une proportion en volume de 2,5 à 25%, de préférence d'au moins 15%.

3. Milieu de culture de mycobactéries selon la revendication 2, **caractérisé en ce que** le colorant est un colorant rouge, dans une concentration de 10 à 100mg/L.

4. Milieu de culture de mycobactéries selon la revendication 3, **caractérisé en ce que** le colorant est choisi parmi l'azurobine et le rouge de Ponceau 4R de préférence dans une concentration de 30 à 50mg/L chacun.

5. Milieu de culture de mycobactéries selon la revendication 4, **caractérisé en ce que** le colorant est un mélange d'azurobine et de rouge de Ponceau 4R de préférence dans une proportion en concentrations de 50/30.

6. Milieu de culture de mycobactéries selon l'une des revendications 1 à 5 **caractérisé en ce qu'**il contient au moins un composé antioxydant, de préférence l'acide ascorbique, de préférence à une concentration d'au moins 0.1g/L.

7. Milieu de culture de mycobactéries selon l'une des revendications 1 à 6 dans lequel la lécithine est comprise dans une proportion pondérale de 0,1 à 5%, de préférence 0,5 à 1%, de préférence la lécithine étant de la lécithine de jaune d'oeuf.

8. Milieu de culture de mycobactéries selon l'une des revendications 1 à 7, **caractérisé en ce qu'**il comprend un milieu de culture de mycobactéries de base comprenant, outre de l'eau distillée, les composants suivants : sulfate d'ammonium, sulfate de magnésium, sulfate de cuivre, sulfate de zinc, citrate de sodium, citrate d'ammonium ferrique, chlorure de sodium, chlorure de calcium, phosphate monopotassique, phosphate disodique, acide L-glutamique, biotine et pyridoxine.

9. Milieu de culture de mycobactéries selon l'une des revendications 1 à 8, **caractérisé en ce que** lesdits antibiotiques sans activité antimycobactérienne sont la polymyxine, amphotéricine B, acide nalidixique, triméthoprime, azlocilline et vancomycine, et le milieu de culture comporte, en outre, un antifongique, de préférence l'amphotéricine B.

10. Milieu de culture de mycobactéries selon l'une des revendications 1 à 9, **caractérisé en ce que** ledit milieu de culture est un milieu de culture solide contenant un produit gélifiant choisi de préférence parmi les géloses et agar, de préférence dans une proportion pondérale de 0.5 à 5%, de préférence encore de 1 à 2%.

11. Milieu de culture de mycobactéries selon la revendication 10, **caractérisé en ce qu'**il s'agit d'un milieu solide de type Middlebrook de référence 7H10, additionné de dit produit gélifiant et des facteurs de croissance de mycobactéries additionnels suivants : acide oléique, albumine bovine, de préférence la fraction V de l'albumine bovine, dextrose, catalase et glycérol.

12. Procédé de culture d'une mycobactérie à l'aide d'un milieu de culture de mycobactéries selon l'une des revendications 1 à 11, **caractérisé en ce que** l'on chauffe un échantillon de prélèvement clinique contenant une dite mycobactérie, dans un dit milieu de culture de mycobactéries, à une température de 30°C à 37°C, appropriée pour la culture de l'espèce de mycobactérie contenue dans l'échantillon.

13. Procédé de culture d'une mycobactérie selon la revendication 12, **caractérisé en ce que** l'on cultive un échantillon de prélèvement clinique contenant une bactérie du complexe *Mycobacterium tuberculosis* à une température de 37°C dans un dit milieu de culture de mycobactéries.

14. Procédé de culture d'une mycobactérie selon la revendication 12 ou 13, **caractérisé en ce que** l'on réalise les étapes suivantes:
- on effectue la culture d'un échantillon de prélèvement clinique pouvant contenir des mycobactéries jusqu'à ce qu'une croissance de bactéries soit détectable, et
- on identifie que la bactérie détectée est une bactérie du genre mycobactérie par un test de coloration, et
- le cas échéant, on identifie l'espèce de ladite mycobactérie par des moyens d'analyse moléculaire, de préférence par spectrométrie de masse.

15. Procédé de culture d'une mycobactérie selon l'une des revendications 12 à 14, dans lequel on réalise une culture d'un dit échantillon de prélèvement clinique pouvant contenir une mycobactérie et des bactéries contaminantes dont la croissance peut inhiber la croissance des mycobactéries, **caractérisé en ce que** :
1/ on réalise une étape préalable de décontamination initiale dudit échantillon dans ledit milieu de culture avec de la chlorhexidine pendant une période limitée, afin de limiter l'action de la chlorhexidine à une activité contre les bactéries autres que mycobactéries, de préférence environ 15 minutes de traitement dans une solution de chlorhexidine à 1% sous agitation, et
2/ on élimine la chlorhexidine en lavant l'échantillon ainsi traité de l'étape 1/ avec un tampon neutre, et on centrifuge et récupère le culot bactérien contenant les bactéries contaminantes ainsi inactivées et les mycobactéries non inactivées, que l'on inocule sur un dit milieu de culture de mycobactéries.

16. Procédé de culture d'une mycobactérie selon l'une des revendications 12 à 15, **caractérisé en ce que** l'on réalise une culture d'un dit échantillon de prélèvement de selles.

17. Procédé de culture d'une mycobactérie selon l'une des revendications 12 à 16, **caractérisé en ce que** ledit milieu de culture est un milieu de culture solide et on détecte une croissance de dites mycobactéries à l'oeil nu lorsque l'on peut apercevoir la formation d'une colonie de bactéries sur ledit milieu de culture solide.

18. Procédé de culture d'une mycobactérie selon la revendication 17, dans laquelle on met en oeuvre un milieu de culture solide, **caractérisé en ce que** l'on réalise les étapes dans lesquelles :
i/- on réalise une culture d'une dite mycobactérie, de préférence du complexe *Mycobacterium tuberculosis,* sur un dit milieu de culture solide en présence d'au moins un antibiotique donné, à différentes concentrations connues, et
ii/- on détermine la plus petite concentration d'antibiotique, de préférence choisi parmi la rifampicine, isoniazide, éthambutol, pyrazinamide et la streptomycine, qui inhibe toute croissance visible de ladite bactérie.

19. Procédé de culture d'une mycobactérie selon la revendication 18, **caractérisé en ce qu'**à l'étape i/-, on dépose, sur ledit milieu de culture solide, au moins une bandelette de papier imprégné d'un dit antibiotique à différentes concentrations selon un gradient de concentration le long de ladite bandelette.

20. Procédé de détection de la croissance d'une bactérie du complexe *Mycrobactérium tuberculosis* en moins de 15 jours, de préférence pas plus de 10 jours permettant la détection de 50% des mycobactéries en moins d'une semaine **caractérisé en ce qu'**on met en oeuvre un procédé de culture selon l'une des revendications 12 à 19.

## Patentansprüche

1. Kulturmedium für Mykobakterien, das in der Lage ist, das Kultivieren von Mykobakterien aus einer klinischen Probe zu ermöglichen, umfassend Lecithin, mykobakterielle Wachstumsfaktoren und vorzugsweise Antibiotika, die gegenüber Mykobakterien nicht aktiv sind, **dadurch gekennzeichnet, dass** es kein Blut umfasst und ferner mindestens die folgenden zusätzlichen Komponenten umfasst:
- Lammserum, vorzugsweise zersetztes Lammserum, und
- mindestens einen roten Farbstoff ohne Aktivität gegen die Mykobakterien, der es erlaubt, die Farbe des Kulturmediums in Kontrast zu den Mykobakterienkolonien zu setzen.

2. Kulturmedium für Mykobakterien nach Anspruch 1, **dadurch gekennzeichnet, dass** das Lammserum in einem Volumenanteil von 2,5 bis 25 %, vorzugsweise mindestens 15 %, enthalten ist.

3. Kulturmedium für Mykobakterien nach Anspruch 2, **dadurch gekennzeichnet, dass** es sich bei dem Farbstoff um einen roten Farbstoff in einer Konzentration von 10 bis 100 mg/l handelt.

4. Kulturmedium für Mykobakterien nach Anspruch 3, **dadurch gekennzeichnet, dass** der Farbstoff ausgewählt ist aus Azurobin und Cochenillerot A, vorzugsweise in einer Konzentration von jeweils 30 bis 50 mg/l.

5. Kulturmedium für Mykobakterien nach Anspruch 4, **dadurch gekennzeichnet, dass** es sich bei dem Farbstoff um eine Mischung aus Azurobin und Cochenillerot A handelt, vorzugsweise in einem Konzentrationsverhältnis von 50/30.

6. Kulturmedium für Mykobakterien nach einem der Ansprüche 1 bis 5, **dadurch gekennzeichnet, dass** es mindestens eine antioxidative Verbindung, vorzugsweise Ascorbinsäure, vorzugsweise in einer Konzentration von mindestens 0,1 g/l enthält.

7. Kulturmedium für Mykobakterien nach einem der Ansprüche 1 bis 6, wobei das Lecithin in einem Gewichtsanteil von 0,1 bis 5 %, vorzugsweise 0,5 bis 1 % vorliegt, wobei es sich bei dem Lecithin vorzugsweise um Eigelb-Lecithin handelt.

8. Kulturmedium für Mykobakterien nach einem der Ansprüche 1 bis 7, **dadurch gekennzeichnet, dass** es ein basisches Kulturmedium für Mykobakterien umfasst, das zusätzlich zu destilliertem Wasser die folgenden Komponenten umfasst: Ammoniumsulfat, Magnesiumsulfat, Kupfersulfat, Zinksulfat, Natriumcitrat, Eisenammoniumcitrat, Natriumchlorid, Calciumchlorid, Monokaliumphosphat, Dinatriumphosphat, L-Glutaminsäure, Biotin und Pyridoxin.

9. Kulturmedium für Mykobakterien nach einem der Ansprüche 1 bis 8, **dadurch gekennzeichnet, dass** es sich bei den Antibiotika ohne antimykobakterielle Aktivität um Polymyxin, Amphotericin B, Nalidixinsäure, Trimethoprim, Azlocillin und Vancomycin handelt, und das Kulturmedium ferner ein Antimykotikum umfasst, vorzugsweise Amphotericin B.

10. Kulturmedium für Mykobakterien nach einem der Ansprüche 1 bis 9, **dadurch gekennzeichnet, dass** das Kulturmedium ein festes Kulturmedium ist, das ein Geliermittel enthält, das vorzugsweise aus Gelosen und Agar ausgewählt ist, vorzugsweise in einem Gewichtsverhältnis von 0,5 bis 5 %, noch bevorzugter 1 bis 2%.

11. Kulturmedium für Mykobakterien nach Anspruch 10, **dadurch gekennzeichnet, dass** es sich um ein festes Medium des Typs Middlebrook mit der Referenz 7H10 handelt, dem das Geliermittel und die folgenden zusätzlichen mykobakteriellen Wachstumsfaktoren zugesetzt sind: Ölsäure, Rinderalbumin, vorzugsweise Rinderalbumin-Fraktion V, Dextrose, Katalase und Glycerin.

12. Verfahren zum Kultivieren eines Mykobakteriums unter Verwendung eines Kulturmediums für Mykobakterien nach einem der Ansprüche 1 bis 11, **dadurch gekennzeichnet, dass** eine das Mykobakterium enthaltende klinische Probe in dem Kulturmedium für Mykobakterien auf eine Temperatur von 30 bis 37 °C erhitzt wird, die zur Kultivierung der in der Probe enthaltenen Mykobakterienarten geeignet ist.

13. Verfahren zum Kultivieren eines Mykobakteriums nach Anspruch 12, **dadurch gekennzeichnet, dass** eine klinische Probe, die ein Bakterium des *Mycobacterium tuberculosis*-Komplexes enthält, bei einer Temperatur von 37 °C in einem der genannten Kulturmedien für Mykobakterien kultiviert wird.

14. Verfahren zum Kultivieren eines Mykobakteriums nach Anspruch 12 oder 13, **dadurch gekennzeichnet, dass** die folgenden Schritte durchgeführt werden:
- Kultivieren einer klinischen Probe, die Mykobakterien enthalten kann, bis ein Bakterienwachstum nachweisbar ist, und
- Identifizieren, ob es sich bei dem nachgewiesenen Bakterium um ein Bakterium der Gattung Mykobakterium handelt, durch einen Färbetest, und
- gegebenenfalls Identifizieren der Spezies des Mykobakteriums durch molekulare Analyse, vorzugsweise durch Massenspektrometrie.

15. Verfahren zum Kultivieren eines Mykobakteriums nach einem der Ansprüche 12 bis 14, wobei eine Kultur einer besagten klinischen Probe erfolgt, die ein Mykobakterium und verunreinigende Bakterien enthalten kann, deren Wachstum das Wachstum der Mykobakterien hemmen kann, **dadurch gekennzeichnet, dass**:
1/ ein vorbereitender Schritt der Erstdekontamination der Probe in dem Kulturmedium mit Chlorhexidin für einen begrenzten Zeitraum durchgeführt wird, um die Wirkung von Chlorhexidin auf eine Aktivität gegen andere Bakterien als Mykobakterien zu beschränken, vorzugsweise etwa 15 Minuten Behandlung in einer 1-%igen Chlorhexidinlösung unter Bewegung, und
2/ Chlorhexidin durch Waschen der so behandelten Probe aus Schritt 1/ mit einem neutralen Puffer entfernt wird und das Bakterienpellet, das die so inaktivierten verunreinigenden Bakterien und die nicht inaktivierten Mykobakterien enthält, zentrifugiert und gewonnen wird und auf ein solches Kulturmedium für Mykobakterien beimpft wird.

16. Verfahren zum Kultivieren eines Mykobakteriums nach einem der Ansprüche 12 bis 15, **dadurch gekennzeichnet, dass** eine Kultur einer Stuhlprobe durchgeführt wird.

17. Verfahren zum Kultivieren eines Mykobakteriums nach einem der Ansprüche 12 bis 16, **dadurch gekennzeichnet, dass** es sich bei dem Kulturmedium um ein festes Kulturmedium handelt und ein Wachstum der Mykobakterien mit bloßem Auge nachgewiesen wird, wenn die Bildung einer Bakterienkolonie auf dem festen Kulturmedium erkennbar ist.

18. Verfahren zum Kultivieren eines Mykobakteriums nach Anspruch 17, bei dem ein festes Kulturmedium verwendet wird, **dadurch gekennzeichnet, dass** die Schritte durchgeführt werden, in denen:
i/ eine Kultur eines besagten Mykobakteriums, vorzugsweise des *Mycobacterium tuberculosis*-Komplexes, auf einem besagten festen Kulturmedium in Gegenwart von mindestens einem gegebenen Antibiotikum in verschiedenen bekannten Konzentrationen durchgeführt wird, und
ii/ die niedrigste Konzentration an Antibiotikum, vorzugsweise ausgewählt aus Rifampicin, Isoniazid, Ethambutol, Pyrazinamid und Streptomycin, die jedes sichtbare Wachstum der Bakterien hemmt, bestimmt wird.

19. Verfahren zum Kultivieren eines Mykobakteriums nach Anspruch 18, **dadurch gekennzeichnet, dass** in Schritt i/- mindestens ein mit einem besagten Antibiotikum imprägnierter Papierstreifen auf das feste Kulturmedium in unterschiedlichen Konzentrationen gemäß einem Konzentrationsgradienten entlang des Streifens aufgebracht wird.

20. Verfahren zum Nachweis des Wachstums eines Bakteriums des *Mycrobacterium tuberculosis-*Komplexes in weniger als 15 Tagen, vorzugsweise nicht mehr als 10 Tagen, das den Nachweis von 50 % der Mykobakterien in weniger als einer Woche ermöglicht, **dadurch gekennzeichnet, dass** ein Verfahren zum Kultivieren gemäß einem der Ansprüche 12 bis 19 durchgeführt wird.

## Claims

1. Mycobacterial culture medium capable of allowing the cultivation of mycobacteria from a clinical sample, comprising lecithin, growth factors of mycobacteria and, preferably, antibiotics not active against mycobacteria, **characterized in that** it does not comprise blood, but further comprises at least the following additional components:
- lamb serum, preferably decomplemented lamb serum, and
- at least one red dye with no activity against mycobacteria, which makes it possible to contrast the color of the culture medium with respect to the colonies of mycobacteria.

2. Mycobacteria culture medium according to claim 1, **characterized in that** the lamb serum is in a volume proportion of 2.5 to 25%, preferably at least 15%.

3. Mycobacteria culture medium according to claim 2, **characterized in that** the dye is a red dye in a concentration of 10 to 100 mg/L.

4. Mycobacteria culture medium according to claim 3, **characterized in that** the dye is selected from azurobine and Ponceau red 4R, preferably in a concentration of 30 to 50mg/L each.

5. Mycobacteria culture medium according to claim 4, **characterized in that** the dye is a mixture of azurobine and Ponceau red 4R, preferably in a concentration ratio of 50/30.

6. Mycobacteria culture medium according to one of the claims 1 to 5, **characterized in that** it contains at least one antioxidant compound, preferably ascorbic acid, preferably at a concentration of at least 0.1g/L.

7. Mycobacteria culture medium according to one of the claims 1 to 6 wherein the lecithin is in a weight proportion of 0.1 to 5%, preferably 0.5 to 1%, preferably wherein the lecithin is lecithin of egg yolk.

8. Mycobacteria culture medium according to one of the claims 1 to 7, **characterized in that** it comprises a base mycobacteria culture medium comprising, in addition to distilled water, the following components: ammonium sulfate, magnesium sulphate, copper sulphate, zinc sulphate, sodium citrate, ferric ammonium citrate, sodium chloride, calcium chloride, monopotassium phosphate, disodium phosphate, L-glutamic acid, biotin and pyridoxine.

9. Mycobacteria culture medium according to one of the claims 1 to 8, **characterized in that** the antibiotics without antimycobacterial activity are polymyxin, amphotericin B, nalidixic acid, trimethoprim, azlocillin and vancomycin, while the culture medium comprises, in addition, an antifungal agent, preferably amphotericin B.

10. Mycobacteria culture medium according to one of the claims 1 to 9, **characterized in that** the culture medium is a solid culture medium containing a gelling agent preferably selected from agar, preferably in a proportion by weight from 0.5 to 5%, more preferably from 1 to 2%.

11. Mycobacteria culture medium according to claim 10, **characterized in that** it is a Middlebrook solid medium of reference 7H10, supplemented with the gelling product and the following additional growth factors of mycobacteria: oleic acid, bovine albumin, preferably fraction V of bovine albumin, dextrose, catalase and glycerol.

12. Method of culturing a mycobacterium using a mycobacteria culture medium according to one of the claims 1 to 11, **characterized in that** a clinical sample containing the mycobacterium is heated, in a culture medium of mycobacteria, at a temperature of 30°C to 37°C, which is suitable for culturing the species of mycobacterium contained in the sample.

13. Method of culturing a mycobacterium according to claim 12, **characterized in that** a clinical specimen sample containing a bacterium of *Mycobacterium tuberculosis* complex is cultivated at a temperature of 37°C in the culture medium of mycobacteria.

14. Method of culturing a mycobacterium according to claim 12 or 13, **characterized in that** the following steps are carried out:
- culturing a clinical specimen that may contain mycobacteria until a growth of bacteria may be detected, and
- the detected bacterium is identified as a bacterium of the mycobacterium genus by a staining test, and
- where appropriate, the species of the mycobacterium is identified by means of molecular analysis, preferably by mass spectrometry.

15. Method of culturing a mycobacterium according to one of the claims 12 to 14, wherein the culturing of the clinical sample may contain a mycobacterium and contaminating bacteria, whose growth may inhibit the growth of mycobacteria, **characterized in that**:
1/ a preliminary step of initial decontamination of the sample in the culture medium is carried out with chlorhexidine for a limited period, in order to limit the action of chlorhexidine to an activity against bacteria other than mycobacteria, preferably about 15 minutes of treatment in a solution of 1% chlorhexidine with stirring, and
2/ the chlorhexidine is removed by washing the sample thus treated in step 1/ with a neutral buffer, and the bacterial pellet containing the contaminating bacteria thus inactivated, as well as the non-inactivated mycobacteria which are inoculated on a so-called mycobacteria culture medium, is recovered.

16. Method of culturing a mycobacterium according to one of the claims 12 to 15, **characterized in that** culturing of a stool sample is carried out.

17. Method of culturing a mycobacterium according to one of the claims 12 to 16, **characterized in that** the culture medium is a solid culture medium and growth of the mycobacteria may be detected by the naked eye, wherein the formation of a colony of bacteria on the solid culture medium may be perceived.

18. Method of culturing a mycobacterium according to claim 17, wherein a solid culture medium is used, **characterized in that** the following steps are carried out:
i/ culturing of a the mycobacterium, preferably of the *Mycobacterium tuberculosis* complex, is carried out on the solid culture medium in the presence of at least one given antibiotic, at various known concentrations, and
ii/ the smallest concentration of antibiotic is determined, preferably selected from rifampicin, isoniazid, ethambutol, pyrazinamide and streptomycin, which inhibits any visible growth of the bacterium.

19. Method of culturing a mycobacterium according to claim 18, **characterized in that**, in step i/, at least one paper test strip impregnated with the antibiotic at different concentrations according to a concentration gradient along the teat strip, is deposited on the solid culture medium.

20. Method for detecting the growth of a *Mycrobacterium tuberculosis* complex bacterium in less than 15 days, preferably not more than 10 days, which enables the detection of 50% of the mycobacteria in less than a week, **characterized in that** a culture method according to one of the claims 12 to 19 is implemented.
